# EUROPÄISCHE PATENTSCHRIFT

(11) **EP 4 378 393 B1**
(45) Veröffentlichungstag und Bekanntmachung des Hinweises auf die Patenterteilung: **20.05.2026**
(21) Anmeldenummer: 22210596.7
(22) Anmeldetag: 30.11.2022
(51) Int. Cl.: A61B 6/00, A61B 18/04, A61B 18/00, A61B 34/00, A61B 34/20, A61B 90/00, A61B 18/18

(54) **SYSTEM ZUR BESTIMMUNG EINER TEMPERATUR UND/ODER TEMPERATURVERTEILUNG IN EINEM ABLATIONSBEREICH**
SYSTEM FOR DETERMINING A TEMPERATURE AND/OR TEMPERATURE DISTRIBUTION IN AN ABLATION REGION
SYSTÈME POUR DÉTERMINER UNE TEMPÉRATURE ET/OU UNE DISTRIBUTION DE TEMPÉRATURE DANS UNE ZONE D'ABLATION

(43) Veröffentlichungstag der Anmeldung: 05.06.2024
(73) Patentinhaber: Siemens Healthineers AG, 91301 Forchheim (DE)
(72) Erfinder: Joy, Vaisakh Nappady, 91052 Erlangen (DE); Kratzke, Lisa, 91054 Erlangen (DE); Noack, Olivia, 95444 Bayreuth (DE); Schäfer, Sebastian, 96146 Altendorf (DE)
(74) Vertreter: Siemens Healthineers Patent Attorneys

(56) Entgegenhaltungen:
- DE-A1- 102008 049 604
- US-A1- 2010 185 087
- SHAPIRA NADAV ET AL: "Non-invasive real-time thermometry via spectral CT physical density quantifications", PROCEEDINGS OF THE SPIE, SPIE, US, vol. 12304, 18 October 2022 (2022-10-18), pages 1230404 - 1230404, XP060166589, ISSN: 0277-786X, ISBN: 978-1-5106-5738-0, DOI: 10.1117/12.2647018

## Beschreibung

Die vorliegende Offenbarung betrifft ein Verfahren zur Bestimmung einer Temperatur und/oder einer Temperaturverteilung in einem Ablationsbereich. Ferner betrifft die Offenbarung ein System, eine Ablationsvorrichtung und ein Computerprogrammprodukt.

Perkutane ablative Verfahren zielen auf die Behandlung lokaler Tumore in festen Organen des Körpers ab. Während des Verfahrens führt der Arzt unter CT- oder US-Anleitung eine Sonde durch die Haut des Patienten in das Zielorgan (z. B. Leber) und das Ziel (z. B. HCC, CRM) ein. Anschließend wird entweder Hitze oder Kälte angewendet, um das Gewebe zu zerstören. Bei der Hitzeanwendung erzeugen die neuesten Systeme ein lokales Mikrowellenfeld, um das Gewebe in einem Radius von bis zu 5 cm zu erhitzen.

Im Bereich der medizinischen Technik ist es bei bestimmten Anwendungen, insbesondere bei Ablationsanwendungen, erforderlich, die Temperatur an interessierenden Körperstellen oder in interessierenden Körperbereichen zu erfassen. Hierfür könnte man zum Beispiel eine Messsonde in den Patienten einführen, die an der entsprechenden Stelle positioniert wird. Damit kann jedoch nur die Temperatur an einer Position bestimmt werden, nicht jedoch eine Temperaturverteilung oder Temperaturkarte, wie sie bspw. für die thermische Tumorablation, insbesondere die Radiofrequenzablation, die laserinduzierte Thermotherapie oder die Kryotherapie, im Rahmen einer medizinischen Anwendung wünschenswert ist.

Grundsätzlich kann die Temperatur auch durch Aufzeichnung eines Bilddatensatzes mit einem Röntgen-Computertomographen bestimmt werden, da die Temperatur mit der Materialdichte korreliert ist, die wiederum die Röntgenabsorption beeinflusst.

So führt z. B. eine Erhöhung der Temperatur im lebenden Körpergewebe zu einer Absenkung der Dichte und somit der Röntgenabsorption in reproduzierbarer Weise. Allerdings ist eine derartige Temperaturbestimmung bei gleichzeitiger Anwesenheit eines weiteren Materials mit veränderlicher Konzentration, wie bspw. Kontrastmittel, nicht mehr möglich. Eine Temperaturbestimmung basierend auf CT-Aufnahmen erlaubt bisher auch nur die Ermittlung einer relativen Temperatur, insbesondere ist die Ermittlung einer absoluten Temperatur im Ablationsbereich basierend auf CT-Aufnahmen nicht möglich.

Auch eine Modellierung basierend auf tabellierten Parametern erlaubt keine zufriedenstellende Temperaturbestimmung für einen Ablationsbereich. Aufgrund der individuellen Physiologie des Patienten lassen sich Umfang und Form einer erzeugten Ablationszone nicht allein durch mathematische Modelle vorhersagen. Die Ablationsvolumina weichen in der Regel um bis zu 20 % (~1 cm) von den Ex-vivo-Messungen, der aktuellen Form der Ablationsmodellierung, ab und können erst nach Abschluss des Verfahrens beurteilt werden. Derzeit ist der Arzt während einer laufenden Ablation so gut wie blind und muss sich vollständig auf die aus Ex-vivo-Modellen gewonnenen thermischen Modelle verlassen.

Als relevanter Stand der Technik sind die Druckschrift DE 10 2008 049604 A1 zu nennen, sowie die wissenschaftliche Publikation "Non-invasive real-time thermometry via spectral CTphysical density quantifications" von SHAPIRA NADA V ET AL (PROCEEDINGS OF THE SPIE, SPIE, US, Bd. 12304, 18. Oktober 2022 (2022-10-18), Seiten 1230404-1230404, XP060166589, ISSN: 0277-786X, 001: 10. 1117/12.2647018, ISBN: 978-1-5106-5738-0)

Die Aufgabe der vorliegenden Offenbarung besteht darin, ein Verfahren zur Bestimmung einer Temperatur und/oder Temperaturverteilung in einem Ablationsbereich anzugeben, wobei die Temperatur und/oder Temperaturverteilung basierend auf Bilddaten erfolgt, wobei die bestimmte Temperatur und/oder Temperaturverteilung insbesondere eine absolute Temperatur ist.

Die Aufgabe wird mit der Ablationsanordnung nach Anspruch 12 sowie dem Computerprogrammprodukt umfassend Programmcodemittel um die Ablationsanordnung dazu zu veranlassen ein Verfahren zur Bestimmung einer Temperatur und / oder einer Temperaturverteilung in einem Ablationsbereich auszuführen nach Anspruch 1, gelöst.

Unabhängig vom grammatikalischen Geschlecht eines bestimmten Begriffes sind Personen mit männlicher, weiblicher oder anderer Geschlechteridentität mit umfasst.

Einen Gegenstand der Offenbarung bildet ein Verfahren zur Bestimmung einer Temperatur und/oder einer Temperaturverteilung in einem Ablationsbereich, umfassend die Schritte:
- Bereitstellen eines ersten Bilddatensatzes eines Objektbereichs, wobei der erste Bilddatensatz bei unterschiedlicher spektraler Verteilung einer Röntgenstrahlung aufgenommene medizinische Bilddaten umfasst, wobei der Objektbereich den Ablationsbereich umfasst,
- Ermitteln einer ersten Dichte, einer ersten Dichteverteilung, eines ersten Schwächungswertes, einer ersten Schwächungswertverteilung und/oder einer ersten Gewebeverteilung in mindestens einem Abschnitt des Objektbereichs basierend auf dem ersten Bilddatensatz,
- Bestimmen einer ersten Sensorposition im Objektbereich basierend auf dem ersten Bilddatensatz,
- Bereitstellen einer ersten Temperatur für die erste Sensorposition,
- Bereitstellen eines zweiten Bilddatensatzes des Objektbereichs, wobei der zweite Bilddatensatz bei unterschiedlicher spektraler Verteilung einer Röntgenstrahlung aufgenommene medizinische Bilddaten umfasst,
- Bestimmen einer zweiten Sensorposition im Objektbereich basierend auf dem zweiten Bilddatensatz,
- Bereitstellen einer zweiten Temperatur für die zweite Sensorposition,
- Ermitteln einer zweiten Dichte, einer zweiten Dichteverteilung, eines zweiten Schwächungswerts, einer zweiten Schwächungswertverteilung und/oder einer zweiten Gewebeverteilung in einem Umgebungsbereich der zweiten Sensorposition basierend auf dem zweiten Bilddatensatz,
- Insbesondere Bestimmen einer Dichte, eines Schwächungswerts, einer Dichteverteilung und/oder Schwächungswertverteilung im Ablationsbereich, vorzugsweise basierend auf dem zweiten, dritten oder weiterem Bilddatensatz,
- Bestimmen einer Temperatur und/oder einer Temperaturverteilung im Ablationsbereich basierend auf der ersten Temperatur, der ersten Sensorposition, der zweiten Temperatur, der zweiten Sensorposition, der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung, vorzugsweise erfolgt das Bestimmen der Temperatur und/oder der Temperaturverteilung basierend auf der ermittelten Dichte, Schwächungswert, Schwächungswertverteilung und/oder Dichteverteilung im Ablationsbereich,
- Bereitstellen der bestimmten Temperatur und/oder Temperaturverteilung des Ablationsbereichs.

Das Verfahren ist zur Bestimmung und/oder Ermittlung einer Temperatur und/oder einer Temperaturverteilung ausgebildet. Die bestimmte Temperatur ist dabei eine Temperatur in dem Ablationsbereich. Der Temperaturverteilung beschreibt eine Verteilung der Temperatur in dem Ablationsbereich. Die Temperatur und/oder der Temperatur. Die Temperaturverteilung beschreibt eine absolute Temperatur, beispielsweise eine Temperatur in Grad Celsius, Fahrenheit oder Kelvin. Das Verfahren ist insbesondere zur Kalibrierung eines Verfahrens oder Systems zur Bestimmung einer Temperatur oder Temperaturverteilung ausgebildet. Insbesondere ist das Verfahren zur Bestimmung einer auf einer Kalibration basierenden Temperatur und/ oder Temperaturverteilung ausgebildet. Der Ablationsbereich ist insbesondere ein Bereich oder Abschnitt eines Organs, eines Körpers, eines Patienten und/oder eines Gewebes. Der Ablationsbereich ist beispielsweise ein Bereich um eine Ablationssonde. Der Ablationsbereich beschreibt beispielsweise den Bereich, der mittels der Ablation, insbesondere Mikrowellen, thermischen oder grün nur Ablationsbehandelt werden soll. Die Temperatur und/oder die Temperaturverteilung beschreibt vorzugsweise eine Temperatur in einem Gewebe, Organ oder Körper. Die Bestimmung der Temperatur und/oder Temperaturverteilung ist insbesondere zur Bestimmung der Temperatur und/oder Temperaturverteilung vor, während und/oder nach einer thermischen Ablation ausgebildet. Das Verfahren zur Bestimmung der Temperatur und/oder der Temperaturverteilung basiert insbesondere auf Bilddaten, insbesondere auf Röntgen und/oder CT Aufnahmen des Ablationsbereichs. Das Verfahren ist insbesondere zur Anwendung und/oder Ausführung während einer Ablation und/oder Ablationsanwendung ausgebildet und/oder geeignet. Mittels des Verfahrens ist es insbesondere möglich, eine absolute Temperatur und/oder absolute Temperaturverteilung basierend auf dem Bilddatensatz, einer Dichte, Gewebeverteilung und/oder eines Schwächungswertes, beispielsweise HU-Wertes, zu bestimmen.

Der erste Bilddatensatz wird vorzugsweise mittels oder durch ein Interface bereitgestellt. Das Bereitstellen des ersten Bilddatensatzes, im Speziellen auch von weiteren Bilddatensätzen, kann ferner durch einen Datenspeicher, beispielsweise PACS, durch eine bildgebende Modalität, beispielsweise einem Computertomografen, oder eine Cloud erfolgen. Der erste Bilddatensatz umfasst und/oder zeigt einen Objektbereich. Der erste Bilddatensatz ist für einen Patienten aufgenommen, wobei der Objektbereich einen Abschnitt des Körpers des Patienten zeigt und/oder abbildet. Der erste Bilddatensatz kann auch als Referenz- oder Ursprungsbilddatensatz bezeichnet werden. Der erste Bilddatensatz ist beispielsweise vor Beginn der Ablationsbehandlung und/oder der Anwendung von Wärme und/ oder Kälte zu Ablation aufgenommen. Vorzugsweise ist der erste Bilddatensatz aufgenommen, nachdem eine Ablationssondee, Ablationsnadel oder Sensoreinrichtung positioniert wurde und/oder sich diese an einer gewünschten oder geplanten Position im Patienten befinden. Der Objektbereich kann ein zweidimensionale oder ein dreidimensionaler Abschnitt sein. Der erste Bilddatensatz umfasst vorzugsweise 3-D oder 2-D Aufnahmen und/oder Bilddaten des Objektbereichs. Der Objektbereich umfasst den Ablationsbereich. Im Speziellen umfasst und/oder zeigt der Objektbereich ein Organ, einen Tumor oder einen zu behandelnden Bereich im Patienten, insbesondere den Bereich der mittels der Ablationsbehandelt werden soll.

Der erste Bilddatensatz umfasst und/oder bildet vorzugsweise einen mittels Computertomografie aufgenommenen Bilddatensatz. Der erste Bilddatensatz umfasst medizinische Bilddaten, beispielsweise 3-D oder 2-D Bilddaten. Im Speziellen umfassen die medizinischen Bilddaten Schichtbilder und/oder Schnittbilder durch den Objektbereichs, z.B. als Röntgen- oder CT-Bilder. Die der erste Medizinbilddatensatz basiert auf einer Aufnahme des Objektbereichs mit unterschiedlicher spektraler Verteilung einer Röntgenstrahlung. Mit anderen Worten basiert der erste Bilddatensatz auf einer dual energy, multi energy oder spektraler Computertomografie des Patienten und/oder Objektbereichs. Durch die Aufnahme des Objektbereichs mit Röntgenstrahlung unterschiedlicher spektraler Verteilung kann Gewebe, insbesondere basierend auf dessen Dichte und/oder unterschiedlichen Schwächungswerten, differenziert und/oder unterschieden werden. Im Speziellen basiert der erste Bilddatensatz auf einer Röntgenaufnahme und/oder Computertomografie unter Verwendung eines Single Photon Counting Detektor. Im Speziellen umfasst der erste Bilddatensatz, medizinische Bilddaten und/oder Röntgenaufnahmen, die basierend auf Röntgenstrahlung unterschiedlicher spektraler Verteilung und/oder Energien aufgenommen wurden.

Der Schritt des Ermittelns einer ersten Dichte, Dichteverteilung, Schwächungswertes, Schwächungswertverteilung und/oder ersten Gewebeverteilung wird im Folgenden auch kurz als erster Ermittlungsschritt bezeichnet. Der erste Ermittlungsschritt basiert vorzugsweise auf einer Bildauswertung und/ oder Bildanalyse. Insbesondere kann der erste Ermittlungsschritt auf der Anwendung eines neuronalen Netzes, eines maschinellen Lernens und vorzugsweise eines Deep Learning Algorithmus. Das Ermitteln im ersten Ermittlungsschritt umfasst insbesondere eine Bildauswertung, beispielsweise eine Segmentierung, Clusternahrung und/oder Klassifizierung von Bereichen und/oder Abschnitten in den medizinischen Bilddaten bzw. den ersten Bilddatensatz. Beispielsweise werden Bereiche hinsichtlich ihrer Schwächungswerte, dichten und/oder Grauwerte segmentiert und/oder geclustert. Beispielsweise wird Bereichen mit gleichem Schwächungswert bzw. Grauwert ein gemeinsamer oder gleicher Dichtewert zugeordnet. Die im ersten Ermittlungsschritt ermittelten Dichten sind insbesondere relative Dichten. Im Speziellen kann der erste Ermittlungsschritt unter Berücksichtigung eines bereitgestellten Atlas oder weiterer medizinische Bilddaten, beispielsweise MR Daten, und/ oder einer Bildregistrierung erfolgen. Beispielsweise wird im ersten Ermittlungsschritt ein Organ segmentiert und/oder detektiert, wobei für dieses Organ eine Dichte oder durchschnittliche Dichte aus der Literatur bekannt ist, sodass die im ersten Ermittlungsschritt ermittelten dichten als absolute Dichten ermittelt werden können. Die im ersten Ermittlungsschritt ermittelten Werte, insbesondere dichten, Schwächungswerte und/oder Verteilungen, sind für mindestens einen Abschnitt des Objektbereichs ermittelt. Im Speziellen werden die im ersten Ermittlungsschritt ermittelten Werte und/oder Verteilungen für den Ablationsbereich und/oder eine Umgebung des Ablationsbereichs ermittelt und/oder bestimmt. Vorzugsweise werden die im ersten Ermittlungsschritt ermittelten Werte und/oder Verteilungen für eine erste, zweite oder weitere Sensorposition ermittelt und/oder bestimmt.

Die erste Sensorposition im Objektbereich wird basierend auf dem ersten Bilddatensatz bestimmt und/oder ermittelt. Insbesondere erfolgt das Bestimmen der ersten Sensorposition basierend auf einer Bildauswertung und/oder Bildanalyse, insbesondere kann dieses Bestimmen zusammen mit dem Schritt des ersten Ermittelns, dem ersten Ermittlungsschritt, erfolgen und einen gemeinsamen Schritt bilden. Die erste Sensorposition ist insbesondere eine Position im Bild bzw. in Koordinaten der Bilddaten. Beispielsweise kann die Sensorposition relativ zum Ablationsbereich ermittelt und/oder bestimmt werden. Die Sensorposition ist insbesondere eine Position und/oder Lage eines ersten Sensorelements bzw. einer Sensoreinrichtung. Beispielsweise wird die Sensoreinrichtung und/oder das Sensorelement in den medizinischen Bilddaten bzw. dem ersten Bilddatensatz detektiert und/oder segmentiert. Die Bestimmung der ersten Sensorposition kann beispielsweise basierend auf Bildauswertungsalgorithmen, insbesondere der Anwendung von Algorithmen des maschinellen Lernens und/oder Deep Learning erfolgen. Als Sensorposition kann eine absolute Position, beispielsweise in Bild- oder Weltkoordinaten, bestimmt werden. Die Sensorposition kann als Punktkoordinate, Fläche oder Volumen bestimmt und/oder aufgefasst werden.

Das Bereitstellen der ersten Temperatur kann beispielsweise über das Interface, den Speicher, vorzugsweise über das Sensorelement, das System und/oder die Ablationsvorrichtung bzw. die Sensoreinrichtung der Ablationsvorrichtung erfolgen. Die erste Temperatur ist insbesondere eine gemessene Temperatur, gemessen mit einem Temperatursensor. Beispielsweise umfasst die Ablationsnadel und/oder Ablationssonde ein Sensorelement zur Ermittlung der ersten Temperatur. Die erste Temperatur ist somit insbesondere eine im Körper des Patienten bzw. im Objektbereich gemessene absolute Temperatur. Die Temperatur ist beispielsweise in Kelvin, Fahrenheit oder Grad Celsius gemessen und/oder bereitgestellt. Insbesondere kann die erste Temperatur eine Mehrzahl an Temperaturwerten umfassen, beispielsweise dann, wenn die Ablationsnadel und/oder die Sensoreinrichtung eine Mehrzahl an Sensorelementen umfasst, die beispielsweise beabstandet angeordnet sind, sodass für die unterschiedlichen Orte der Sensorelemente unterschiedliche Temperaturwerte ermittelt und bereitgestellt sind. Die bereitgestellte erste Temperatur ist insbesondere eine gemessene Temperatur vor Beginn der Ablation und/oder zu einem Zeitpunkt der Aufnahme des ersten Bilddatensatzes. Mit anderen Worten beschreibt die erste Temperatur die Temperatur bzw. die gemessene Temperatur, die zu dem Zeitpunkt der Aufnahme des ersten Bilddatensatzes an dem an der bestimmten Sensorposition vorlag bzw. gemessen wurde.

Das Bereitstellen des zweiten Bilddatensatzes ist vorzugsweise ausgebildet und/oder durchgeführt, wie für das Aufnehmen des ersten Bilddatensatzes bzw. das Bereitstellen des ersten Bilddatensatzes beschrieben. Der zweite Bilddatensatz basiert ebenfalls auf der Aufnahme des Objektbereichs mit Röntgenstrahlung unterschiedlicher spektraler Verteilung. Mit anderen Worten bildet der zweite Bilddatensatz eine multi energy, dual energy, spektraler Computertomografie des Objektbereichs. Im Speziellen basieren die bereitgestellten medizinischen Bilddaten des zweiten Medizindatensatzes auf der Verwendung eines Single Photon Counting Detektors. Die medizinischen Bilddaten des zweiten Bilddatensatzes sind vorzugsweise basierend auf den gleichen Einstellungen und/oder Parametern aufgenommen, wie die medizinischen Bilddaten des ersten Bilddatensatzes. Mit anderen Worten sind die medizinischen Bilddaten des ersten und des zweiten Bilddatensatzes direkt vergleichbar und/oder Veränderungen durch Vergleich der Bilddaten feststellbar und/oder erkennbar, insbesondere ohne Vorverarbeitung und/oder Bildanpassung. Die medizinischen Bilddaten des zweiten Bilddatensatzes bzw. der zweite Bilddatensatz ist zu einem Zeitpunkt nach der Aufnahme des ersten Bilddatensatzes aufgenommen. Im Speziellen sind die medizinischen Bilddaten des zweiten Bilddatensatzes zu einem Zeitpunkt nach Beginn der thermischen Ablation bzw. der Ablationsbehandlung aufgenommen. Dies basiert auf der Überlegung, dass durch den Beginn der Ablationsbehandlung ein Temperaturanstieg im Objektbereich zu erwarten ist, sodass dieser in den medizinischen Bilddaten sichtbar sein müsste.

Die zweite Sensorposition im Objektbereich wird basierend auf den medizinischen Bilddaten des zweiten Bilddatensatzes bestimmt bzw. ermittelt. Insbesondere erfolgt das Bestimmen der zweiten Sensorposition ähnlich und/oder auf gleiche Art, wie die Bestimmung der ersten Sensorposition. Mit anderen Worten sind die erste Sensorposition und die zweite Sensorposition auf gleiche oder vergleichbare Art und Weise bestimmt, sodass die ermittelten bzw. bestimmten ersten und zweiten Sensorpositionen für einen direkten Vergleich herangezogen werden können. Die zweite Sensorposition entspricht, insbesondere der Position des oder der Sensorelemente während der Aufnahme der medizinischen Bilddaten des zweiten Bilddatensatzes bzw. des zweiten Bilddatensatzes.

Das Bereitstellen der zweiten Temperatur ist insbesondere ausgebildet, wie das Bereitstellen der ersten Temperatur. Die zweite Temperatur wird beispielsweise durch die Sensorelemente aufgenommen und bereitgestellt. Die zweite Temperatur basiert auf der Messung der Temperatur an der zweiten Sensorposition. Die zweite Temperatur ist wiederum insbesondere eine absolute Temperatur in Kelvin, Grad Celsius oder Fahrenheit. Die bereitgestellte zweite Temperatur entspricht damit insbesondere einer Temperatur nach Beginn der Ablationsbehandlung.

Der Schritt, Ermitteln einer zweiten Dichte, Dichteverteilung, Schwächungswerts, Schwächungswertverteilung und/oder Gewebeverteilung wird im Folgenden als zweiter Ermittlungsschritt bezeichnet. Der zweite Ermittlungsschritt ist insbesondere ausgebildet und/oder gleich zum Ermitteln der zum ersten Ermittlungsschritt. Im zweiten Ermittlungsschritt werden die Werte und/oder Verteilungen basierend auf dem zweiten Bilddatensatz bzw. den medizinischen Bilddaten des zweiten Bilddatensatzes bestimmt und/oder ermittelt. Insbesondere basiert der zweite Ermittlungsschritt auf einer Bildauswertung und/oder einer Bildanalyse. Die zweite Dichte, Dichteverteilung, Schwächungswert, Schwächungswertverteilung und/oder Gewebeverteilung wird für einen Abschnitt des Objektbereichs oder für den gesamten Objektbereich ermittelt und/oder bestimmt. Insbesondere erfolgt das Ermitteln des zweiten Ermittlungsschrittes für eine Umgebung um die zweite Sensorposition. Dies basiert auf der Überlegung, dass so für die zweite Sensorposition bzw. eine Umgebung der zweiten Sensorposition neben der zweiten Temperatur auch eine Dichte, Dichteverteilung, ein Schwächungswert, eine Schwächung Wertverteilung oder eine Gewebeverteilung bekannt sind. Insbesondere wird im ersten Ermittlungsschritt die Dichte, Dichteverteilung, der Schwächungswert, die Schwächung Wertverteilung und/oder Gewebeverteilung für eine Umgebung um die erste Sensorposition bestimmt und/oder ermittelt.

Das Bestimmen einer Temperatur und/oder einer Temperaturverteilung im Ablationsbereich erfolgt basierend auf den ermittelten Werten und/oder Verteilungen des ersten und des zweiten Ermittlungsschritts sowie auf der bereitgestellten ersten und zweiten Temperatur und der ersten und zweiten Sensorposition. Durch das Bestimmen von Dichte und/oder Schwächungswert zu zwei unterschiedlichen Zeitpunkten, wobei für diese Werte und/oder Verteilungen eine gemessene Temperatur bereitgestellt ist, ist es möglich, weitere Dichten und/oder Schwächungswerte in eine Temperatur umzurechnen. Dies basiert auf der Überlegung, dass Dichte und/oder Schwächungswert, die in medizinischen Bilddaten, welche insbesondere für unterschiedliche spektraler Verteilungen einer Röntgenstrahlung aufgenommen sind, mit der Temperatur korrelieren, insbesondere näherungsweise linearer, sodass eine Beziehung zwischen Dichte bzw. Schwächungswert und Temperatur extrapoliert weist.

Die vorliegende Offenbarung erlaubt es, eine absolute Temperatur in einem Ablationsbereich basierend auf medizinischen Bilddaten, die mittels Röntgenstrahlung unterschiedlicher spektraler Verteilung aufgenommen sind, zu bestimmen. Somit ist es möglich, die absolute Temperatur während eines Ablationsprozesses bzw. einer Ablationsanwendndung im Ablationsbereich basierend auf medizinischen Bilddaten zu überwachen, sodass eine Überhitzung einer Umgebung und/oder eines zu schützenden Bereichs verhindert werden kann und/oder sodass das Erreichen einer mindestens zu erreichenden Ablationstemperatur gewährleistet werden kann.

Besonders bevorzugt ist es, dass im zweiten Ermittlungsschritt eine zweite Dichteverteilung als Verteilung der Dichte oder eine zweite Schwächungswertverteilung als Verteilung des Schwächungswerts für den Objektbereich, insbesondere den gesamten Objektbereich ermittelt wird. Mit anderen Worten wird für den Objektbereich, insbesondere für Flächen oder Raumabschnitte, beispielsweise eines Netzes oder einer Punktwolke, jeweils ein Dichtewert oder ein Schwächungswert ermittelt. Insbesondere wird im zweiten Ermittlungsschritt eine Dichte, Dichteverteilung, Schwächungswert und/oder Schwächungswertverteilung im Ablationsbereich ermittelt. Mit anderen Worten beschreibt die zweite Dichteverteilung eine räumliche Verteilung der Dichte im Objektbereich, wobei die Schwächungswertverteilung eine räumliche Verteilung der Schwächungswerte im Objektbereich beschreibt. Insbesondere kann es vorgesehen sein, dass als Temperaturverteilung die Verteilung bzw. räumliche Verteilung der Temperatur im Objektbereich bestimmt und/oder ermittelt wird. Mit anderen Worten beschreiben die Verteilungen eine Kartierung der Dichte, der Schwächungswerte und/oder der Temperatur. Im Speziellen ist es vorgesehen, dass im ersten Ermittlungsschritt die erste Dichte, die erste Dichteverteilung, der erste Schwächungswert, die erste Schwächungswertverteilung und/oder die Gewebeverteilung in einem Abschnitt und/oder Bereich um die erste Sensorposition ermittelt wird. Dies basiert auf der Überlegung, dass durch die Bestimmung der Werte und/oder Verteilungen in der Umgebung um die erste Sensorposition zur on die erste Temperatur einer Dichte oder Schwächungswert zugeordnet werden kann.

Besonders bevorzugt ist es, dass basierend auf dem ersten Bilddatensatz und dem zweiten Bilddatensatz, im Speziellen basierend auf den Dichten, Schwächungswerten bzw. deren Verteilungen, welche im ersten bzw. zweiten Ermittlungsschritt ermittelt wurden, eine Dichteveränderung, eine Schwächungswertveränderung und/oder eine Gewebeverteilungsveränderung bestimmt wird. Die Dichteveränderung beschreibt, beispielsweise die Veränderung der Dichte, der Dichtewerte und/oder der Dichteverteilung zwischen bzw. bezüglich der ersten und der zweiten Bilddatensätze. Die Schwächungswertveränderung beschreibt die Veränderung des Schwächungswerts, der Schwächungswertverteilung bezüglich dem ersten und dem zweiten Bilddatensatz. Die Gewebeverteilungsveränderung beschreibt die Veränderung der Gewebeverteilung, welche basierend auf, bzw. in dem ersten und dem zweiten Ermittlungsschritt ermittelt wurden. Die Bestimmung der Temperatur und/oder der Temperaturverteilung im Ablationsbereich kann insbesondere basierend auf der ersten Temperatur, der zweiten Temperatur sowie der Dichteveränderung, der Schwächungswertveränderung und/oder der Gewebeveränderung bestimmt und/oder ermittelt werden. Beispielsweise kann die Schwächungswertveränderung mit einer Veränderung der Temperatur korreliert werden. Selbiges kann für Dichtewerte und/oder für die Gewebeverteilungsveränderung zur Bestimmung der Temperatur und/oder Temperaturveränderung angenommen werden. Beispielsweise kann die Schwächung Wertveränderung für eine erste Sensorposition und eine zweite Sensorposition mit räumlichen Überlapp mit der Temperaturänderung zwischen zweiter und erster Temperatur korreliert werden.

Besonders bevorzugt ist es, dass eine Kalibrationsfunktion bestimmt wird. Die Kalibrationsfunktion kann eine analytische Funktion oder Relation bilden. Im Speziellen kann die Kalibrationsfunktion auch als eine nicht-analytische Funktion, beispielsweise tabellarische Funktion oder Look-up Funktion, ausgebildet sein. Die Kalibrationsfunktion kann insbesondere auch eine Funktion bzw. Algorithmus des maschinellen Lernens bilden und/oder darauf basieren. Die Kalibrationsfunktion ist ausgebildet und/oder geeignet, einer Dichte, einer Dichteänderung, einem Schwächungswert und/oder einer Schwächungswertveränderung eine Temperatur zuzuordnen. Die Kalibrationsfunktion ist insbesondere bezüglich der ersten und zweiten Bilddatensätze bestimmt bzw. festgelegt. Mit anderen Worten basiert die Kalibrationsfunktion auf dem ersten und dem zweiten Bilddatensatz bzw. den hierfür verwendeten Bildgebungsmodalitäten, Parametern und/oder Einstellungen. Ferner kann die Kalibrationsfunktion anatomische und/oder patientenspezifische Eigenschaften berücksichtigen, welche sich insbesondere in den medizinischen Bilddaten des ersten und des zweiten Bilddatensatzes widerspiegeln.

Gemäß einer optionalen Ausgestaltung der Erfindung ist es vorgesehen, dass der Schritt des Bereitstellens des ersten und/oder des zweiten Bilddatensatzes eine Artefaktbereinigung umfasst und/oder die bereitgestellten Bilddatensätze auf einer Artefaktbereinigung basieren bzw. artefaktbereinigt sind. Die Artefaktbereinigung ist ausgebildet, Artefakte in den medizinischen Bilddaten, welche beispielsweise basierend auf Metallgegenständen im Objektbereich während der Aufnahme der Bilddatensätze hervorgerufen werden. Beispielsweise kann die Verwendung von metallischen und/oder Metall umfassenden Ablationsnadeln und/oder Sensoreinrichtungen Artefakte in den medizinischen Bilddaten bzw. Bilddatensätzen hervorrufen. Durch die Anwendung der Artefaktbereinigung können so hervorgerufene Artefakte in den medizinischen Bilddaten bereinigt und/ oder verringert werden. Beispielsweise umfasst der Schritt des Bereitstellens des ersten und/oder das zweiten Bilddatensatzes die Anwendung eines Artefaktbereinigungsalgorithmus auf die mittels der Tomografie und/oder der Röntgenstrahlung aufgenommenen Bildern. Die artefaktbereinigten medizinischen Bilddaten, bzw. ersten und zweiten Bilddatensätze werden zur Bestimmung der ersten und/oder zweiten Dichte, Dichteverteilung, Schwächungswert, Schwächungswertverteilung, Sensorposition und/oder Gewebeverteilung herangezogen.

Das Bereitstellen der ersten Temperatur und/oder der zweiten Temperatur erfolgt durch eine Sensoreinrichtung. Die Sensoreinrichtung umfasst mindestens ein erstes Sensorelement, sowie ein zweites und/oder weitere Sensorelemente. Die Sensorelemente sind ausgebildet, eine Temperatur zu erfassen und/oder zu bestimmen.

Die Sensorelemente sind als Temperatursensoren ausgebildet.

Das erste und das zweite, insbesondere die weiteren Sensorelemente sind beabstandet zueinander, in einem festen Sensorabstand und/oder äquidistant angeordnet. Beispielsweise sind die Sensorelemente entlang einer gemeinsamen Achse, welche beispielsweise durch eine Längserstreckung der Ablationsnadel gegeben sein kann, angeordnet.

Bei der Bestimmung der ersten Sensorposition erfolgt insbesondere eine Bestimmung einer ersten Sensorelementposition, einer zweiten Sensorelementposition und/oder weitere Sensorelementpositionen. Mit anderen Worten umfasst die bestimmte erste Sensorposition eine Mehrzahl an Sensorelementpositionen. Ebenso kann es vorgesehen sein, dass die Bestimmung der zweiten Sensorposition. Die Bestimmung einer ersten Sensorelementposition, einer zweiten Sensorelementposition und/oder weitere Sensorelementpositionen umfasst. Beispielsweise werden als Sensorpositionen Zahlen-Tupel bereitgestellt, welche die jeweiligen Sensorelementpositionen umfassen. Bei dieser Ausgestaltung ist es ferner vorgesehen, dass die bereitgestellte erste Temperatur und/oder zweite Temperatur jeweils einen ersten Temperaturwert für die Sensorelementposition umfassen, eine zweiten Temperaturwert für die zweite Sensorelementposition und/oder weitere Temperaturwerte für weitere Sensorelementpositionen umfassen. Diese Ausgestaltung basiert auf die Überlegung, durch die Verwendung einer Sensoreinrichtung mit mehreren Sensorelementen eine Bereitstellung von mehreren Temperaturwerten für unterschiedliche Sensorelementpositionen zu ermöglichen, sodass für basierend auf den ersten bzw. zweiten Bilddatensatz bestimmte Sensorpositionen mehrere messbare von Temperaturwerten und Sensorelementpositionen bestimmt werden können. Dies erlaubt insbesondere eine genauere Temperaturbestimmung basierend auf medizinischen Bilddaten.

Besonders bevorzugt ist es vorgesehen, dass die Sensoreinrichtung von einer Ablationsvorrichtung umfasst ist. Die Ablationsvorrichtung umfasst und/oder bildet beispielsweise eine Ablationsnadel und/oder Ablationssondeen. Die Sensorelemente der Sensoreinrichtung, welche von der Ablationsnadel umfasst ist, sind vorzugsweise entlang der Längsachse der Ablationsnadel angeordnet. Die Ablationsvorrichtung, insbesondere die Ablationsnadel, umfasst einen Ablationsabschnitt. Der Ablationsabschnitt ist vorzugsweise in einem Endbereich der Ablationsnadel bzw. der Ablationsvorrichtung angeordnet. Der Ablationsabschnitt ist ausgebildet, eine thermische Ablation in seiner Umgebung durchzuführen und/oder anzuwenden. Beispielsweise ist im Ablationsabschnitt ein Mikrowellengenerator zur Mikrowellen Anwendung und/oder thermischen Behandlung der Umgebung angeordnet. Alternativ und/oder ergänzend kann im Ablationsabschnitt ein Heizelement und/oder ein Kühlelement angeordnet sein. Die Sensoreinrichtung und/oder die Sensorelemente sind insbesondere beabstandet vom Ablationsabschnitt angeordnet, vorzugsweise beträgt der Abstand zwischen Ablationsabschnitt und Sensorelement mindestens 1 cm, vorzugsweise mindestens 5 cm. Durch die Beabstandung von Sensorelement und Ablationsabschnitt können insbesondere empfindliche Sensorelemente angewendet und/oder verwendet werden, insbesondere die Lebensdauer solcher Sensorelemente gesteigert werden.

Vorzugsweise ist es vorgesehen, dass dritte und/oder weitere Bilddatensätze bereitgestellt und/oder aufgenommen werden. Das Bereitstellen des dritten und/oder der weiteren Bilddatensätze erfolgt insbesondere wie das Bereitstellen des ersten und/oder das zweiten Bilddatensatzes. Der dritte und/oder die weiteren Bilddatensätze umfassen medizinische Bilddaten des Objektbereichs, wobei diese wiederum für unterschiedliche spektraler Verteilungen einer Röntgenstrahlung aufgenommen und/oder bereitgestellt wurden. Die dritten und/oder weiteren mit Bilddatensätze sind insbesondere nach den zweiten Bilddatensatz aufgenommen. Beispielsweise kann es vorgesehen sein, dass nach den bereitgestellten zweiten Bilddatensatz in regelmäßigen Abständen weitere Bilddatensätze des Objektbereichs aufgenommen und/oder bereitgestellt werden. Die ersten, zweiten, dritten und/oder weiteren Bilddatensätze bilden insbesondere eine Zeitreihe an medizinischen Bilddaten des Objektbereichs. Diese Ausgestaltung sieht ferner vor, dass eine dritte Temperatur und/oder dritte Temperaturverteilung, im Speziellen eine weitere Temperatur und/oder weitere Temperaturverteilungen im Ablationsbereich, insbesondere im Objektbereich, bestimmt und/oder ermittelt werden. Mit anderen Worten kann so eine Zeitreihe der Temperatur und/oder Temperaturverteilung im Ablationsbereich und/oder im Objektbereich bestimmt und/oder ermittelt werden. Das Bestimmen der dritten Temperatur und/oder Temperaturverteilung, insbesondere der weiteren Temperaturen und/oder Temperaturverteilungen, erfolgt insbesondere basierend auf der ersten Temperatur, der ersten Sensorposition, der zweiten Temperatur, der zweiten Sensorposition und den in dem ersten und zweiten Ermittlungsschritt ermittelten Werten und/oder Verteilungen. Insbesondere ist es nicht nötig, eine dritte Temperatur bzw. eine dritte Sensorposition basierend auf den dritten Bilddatensatz zu bestimmen. Die Bestimmung der dritten und/oder weiteren Temperatur bzw. Temperaturverteilung erfolgt mit anderen Worten basierend auf den medizinischen Bilddaten, bzw. dem dritten Bilddatensatz, und/oder ohne Bestimmung, Messung und/oder Bereitstellung einer Temperatur oder Sensorposition für einen Zeitpunkt nach der Aufnahme des zweiten Bilddatensatzes.

Ferner ist es vorzugsweise vorgesehen, dass basierend auf der ersten, zweiten und dritten Temperatur bzw. Temperaturverteilung ein geplanter Temperaturverlauf, auch extrapoliert der Temperaturverlauf genannt, im Ablationsbereich oder im Objektbereich ermittelt wird. Insbesondere kann das Ermitteln des geplanten Temperaturverlaufs basierend auf der ersten und zweiten Temperatur bzw. Temperaturverteilung sowie dem dritten Bilddatensatz und/oder weiteren Bilddatensätzen erfolgen. Der ermittelte geplante Temperaturverlauf wird anschließend bereitgestellt, beispielsweise einem Anwender und/oder Benutzer angezeigt. Basierend auf dem geplanten Temperaturverlauf kann der Anwender und/oder Nutzer den Verlauf der Ablationsanwendung überwachen und erhält so ein Feedback, wann eine gewünschte Temperatur oder eine zu vermeidende Temperatur erreicht wird.

Besonders bevorzugt ist es, dass basierend auf bereitgestellten Gewebeparametern und/oder Ablationsbetriebsdaten. Der geplante Temperaturverlauf ermittelt wird. Mit anderen Worten kann der geplante Temperaturverlauf unter Zuhilfenahme von Gewebeparameter und/oder Ablationsbetriebsdaten, beispielsweise einer Leistung und/oder Betriebsparametern der Ablationsvorrichtung, genauer bestimmt, extrapoliert und/oder ermittelt werden.

Eine Ausgestaltung des Verfahrens sieht vor, dass basierend auf dem geplanten Temperaturverlauf eine geplante Restablationsdauer ermittelt wird. Die geplante Restablationsdauer ist insbesondere die Zeitdauer, die bis zum Erreichen einer gewünschten und/oder benötigten Temperatur im Ablationsbereich zu rechnen ist und/oder benötigt wird. Die Rest Restablationsdauer kann beispielsweise dem Nutzer und/oder Anwender auf einer Anzeige angezeigt werden, sodass dieser die verbleibende Behandlungsdauer besser abschätzen kann und so eine Schädigung umliegenden Gewebes durch zu lange Ablation oder eine nicht genügende Ablation würden durch zu geringe Restablationsdauer vermeiden kann.

Ferner ist es vorzugsweise vorgesehen, dass basierend auf der ersten Temperatur, der zweiten Temperatur der dritten Temperatur, der ersten Temperaturverteilung, zweiten Temperaturverteilung und/oder dritten Temperaturverteilung ein Wärmeverlust bzw. Wärmeverlustbereiche bestimmt und/oder ermittelt werden. Beispielsweise kann es basierend auf anatomischen Gegebenheiten dazu kommen, dass der Ablationsbereich nicht gleichmäßig erhitzt oder gekühlt wird, beispielsweise durch Materialtransport im Gewebe und/oder Blutbahn, sodass Bereiche des Ablationsbereich nicht gleichlaufend mit dem restlichen Bereich erwärmt oder abgekühlt werden, sodass dieser Bereich unter Umständen nicht genügend behandelt wird. Basierend auf den ermittelten Temperaturen und/oder Temperaturverteilungen sind solche Verlustbereiche bestimmbar. Insbesondere werden die bestimmten Wärmeverlustbereiche dem Anwender und/oder Nutzer angezeigt und/oder bereitgestellt, beispielsweise in den medizinischen Bilddaten angezeigt, sodass der Benutzer und/oder Anwender, gegebenenfalls diese Bereiche separat und/oder zusätzlich eine Ablation unterziehen kann.

Einen weiteren Gegenstand der Erfindung bildet eine Ablationsanordnung umfassend ein System zur Bestimmung der Temperatur und/oder Temperaturverteilung in einem Ablationsbereich.

Das System ist insbesondere ausgebildet und/oder eingerichtet, das obige Verfahren anzuwenden und/oder umzusetzen. Das System zur Bestimmung einer Temperatur und/oder einer Temperaturverteilung in einem Ablationsbereich umfasst ein Bilddatenbereitstellungsmodul, ein Bilddatenauswertemodul, ein Sensordatenbereitstellungsmodul, ein Positionsbestimmungsmodul, ein Temperaturbestimmungsmodul und ein Bereitstellungsmodul, wobei:
- Das Bilddatenbereitstellungsmodul ausgebildet ist, einen ersten Bilddatensatzes eines Objektbereichs bereitzustellen, wobei der erste Bilddatensatz bei unterschiedlicher spektraler Verteilung einer Röntgenstrahlung aufgenommene medizinische Bilddaten umfasst, wobei der Objektbereich den Ablationsbereich umfasst,
- Das Bilddatenauswertemodul ausgebildet ist eine erste Dichte, eine erste Dichteverteilung, einen ersten Schwächungswert, eine erste Schwächungswertverteilung und/oder eine erste Gewebeverteilung in mindestens einem Abschnitt des Objektbereichs basierend auf dem ersten Bilddatensatz zu ermitteln,
- Das Positionsbestimmungsmodul ausgebildet ist, eine erste Sensorposition im Objektbereich basierend auf dem ersten Bilddatensatz zu bestimmen,
- Das Sensordatenbereitstellungsmodul ausgebildet ist, eine erste Temperatur für die erste Sensorposition bereitzustellen,
- Das Bilddatenbereitstellungsmodul ausgebildet ist, einen zweiten Bilddatensatz des Objektbereichs bereitzustellen, wobei der zweite Bilddatensatz bei unterschiedlicher spektraler Verteilung einer Röntgenstrahlung aufgenommene medizinische Bilddaten umfasst,
- Das Positionsbestimmungsmodul ausgebildet ist. eine zweite Sensorposition im Objektbereich basierend auf dem zweiten Bilddatensatz zu bestimmen,
- Das Sensordatenbereitstellungsmodul ausgebildet ist, eine zweite Temperatur für die zweite Sensorposition bereitzustellen,
- Das Bilddatenauswertemodul ausgebildet ist, eine zweite Dichte, eine zweite Dichteverteilung, einen zweiten Schwächungswert, eine zweite Schwächungswertverteilung und/oder eine zweite Gewebeverteilung in einem Umgebungsbereich der zweiten Sensorposition basierend auf dem zweiten Bilddatensatz zu ermitteln,
- Das Temperaturbestimmungsmodul ausgebildet ist, eine Temperatur und/oder eine Temperaturverteilung im Ablationsbereich basierend auf der ersten Temperatur, der ersten Sensorposition, der zweiten Temperatur, der zweiten Sensorposition, der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung zu bestimmen,
- Das Bereitstellungsmodul ausgebildet ist, die bestimmte Temperatur und/oder Temperaturverteilung bereitzustellen.

Die Ablationsanordnung ist zur Ablation eines Gewebes und/oder eines Tumors ausgebildet und/oder eingerichtet. Die Ablationsanordnung umfasst insbesondere eine Ablationsvorrichtung, beispielsweise eine Ablationssonde und/oder eine Ablationsnadel. Die Ablationsanordnung, insbesondere die Ablationsvorrichtung umfasst eine Sensoreinrichtung, wobei die Sensoreinrichtung zur Messung und/oder Bereitstellung der ersten, zweiten und/oder Temperatur ausgebildet und/oder eingerichtet ist. Die Ablationsanordnung, die Ablationsvorrichtung bzw. die Sensoreinrichtung, umfasst insbesondere mindestens einen Sensorelement, wobei der Sensorelement zur Messung und Bereitstellung einer Temperatur ausgebildet und/oder eingerichtet ist.

Einen weiteren Gegenstand der Erfindung bildet ein Computerprogrammprodukt, wobei das Computerprogramm zur aus Führung und/oder Anwendung des obigen Verfahrens ausgebildet und/oder eingerichtet ist.

Weitere vor Teile, Wirkungen und Ausgestaltungen ergeben sich aus den beigefügten Figuren und deren Beschreibung. Dabei zeigen:
- Figur 1: ein Ausführungsbeispiel einer Ablationsvorrichtung;
- Figur 2: beispielhaft einen Bilddatensatz;
- Figur 3: eine Ausführungsbeispiel einer Bereitstellung der bestimmten Temperatur im Ablationsbereich;
- Figur 4: ein Flussdiagram eines Ausführungsbeispiels des Verfahrens zur Bestimmung der Temperatur im Ausführungsbereich.

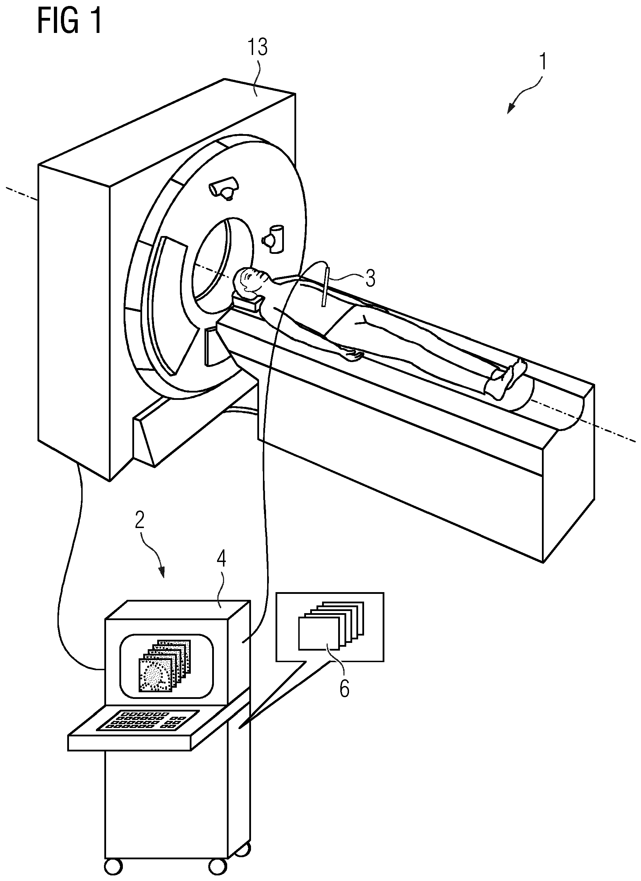

In der Fig. 1 wird beispielhaft eine Ablationsanordnung 1 gezeigt. Die Ablationsanordnung 1 umfasst eine Ablationsvorrichtung 2, wobei die Ablationsvorrichtung 2 eine Ablationssonde 3, auch Ablationsnadel genannt, umfasst. gezeigt. Die Ablationsanordnung 1 umfasst ein System 4 zur Bestimmung einer Temperatur T_{A} und/oder einer Temperaturverteilung T_{A}(x, y, z) in einem Ablationsbereich 5. Der Die Ablationsvorrichtung 2 ist ausgebildet, die Ablationssonde 3 anzusteuern und/oder zu regeln. Die Ablationsvorrichtung 2 versorgt die Ablationssonde 3 beispielsweise mit Strom und/oder regelt bzw. steuert Betriebsparameter. Die Ablationssonde 3 ist ausgebildet, einen Ablationsbereich 5 zu erhitzen, abzukühlen und/oder Mikrowellen zum Erhitzen auszusenden. Das Erhitzen, Vereisen und/oder Aussenden von Mikrowellen bildet eine und/oder ist Teil einer Ablationsanwendung bzw. Ablationsbehandlung.

Das System 1 ist mit einem Computertomographie-System 6 verbunden. Das Computertomographie-System 6, auch kurz Computertomograph genannt, ist zur Aufnahme von medizinischen Bilddaten, insbesondere umfasst von einem Bilddatensatz, ausgebildet. Das Computertomographie-System 6 ist zur Aufnahme der medizinischen Bilddaten mittels Röntgenstrahlung unterschiedlicher spektraler Verteilung, im Speziellen zur Aufnahme von dual energy, multi energy und/oder spektraler CT-Bilder ausgebildet. Im Speziellen umfasst das Computertomographie-System 6 einen photon counting Detektor, insbesondere einen single photon counting detektor. Das Computertomographie-System 6 ist ausgebildet dem System 1 einen ersten Bilddatensatz, einen zweiten Bilddatensatz und im Speziellen einen dritten oder weitere Bilddatensätze 7 bereitzustellen.

Zur Beobachtung einer räumlichen Temperaturänderung in einem vorbestimmten Bereich des Patienten, insbesondere in dem Ablationsbereich 5, die bei der Entfernung eines Tumors durch Hitze entsteht, können sowohl vor der Ablationsbehandlung als auch während der Ablationsbehandlung CT-Aufnahmen erstellt werden und als erste, zweite, dritte und/oder weitere Bilddatensätze 6 bereitgestellt werden.

Es wird darauf hingewiesen, dass unter einem CT-System im Sinne dieser Druckschrift auch ein C-Bogen-System zu verstehe ist, mit welchem ebenso CT-Bilddatensätze erzeugt werden können.

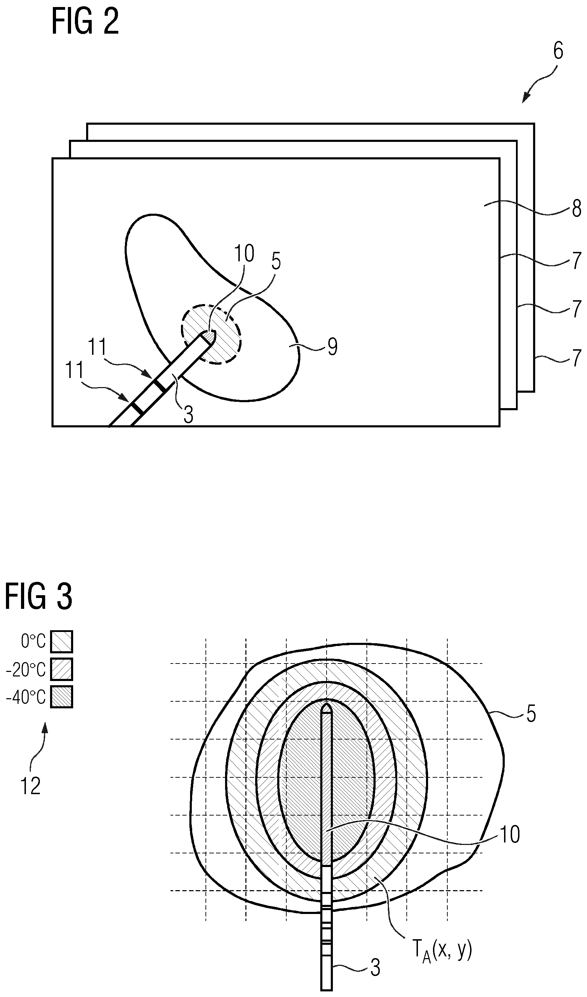

Die Fig. 2 zeigt beispielhaft einen Bilddatensatz 6, z.B. einen ersten Bilddatensatz. Der Bilddatensatz 6 umfasst eine Mehrzahl an medizinischen Bilddaten 7 eines Objektbereichs 8, z.B. Schichtbilder. Die Bilddaten 7 zeigen und/oder Objektbereich 8 umfasst einen Zielbereich, z.B. Organ 9 das der einen Tumor aufweist, wobei der Tumor mittels der Ablationssonde 3 in einer Ablationsbehandlung behandelt werden soll. ein Flussdiagramm des erfindungsgemäßen Verfahrens.

Die Ablationssonde 3 umfasst einen Ablationsabschnitt 10. Der Ablationsabschnitt 10 ist in einem Endbereich der nadel- und/oder stabförmigen Ablationssonde 3 angeordnet. Der Ablationsabschnitt 10 umfasst ein Heizelement, ein Kühlelement und/oder einen Mikrowellengenerator zur Anwendung von Kälte, Hitze oder Mikrowellen in dem Ablationsbereich 5. Der Ablationsbereich 5 ist ein Volumen- und/oder Flächenabschnitt um den Ablationsabschnitt 10.

Die Ablationssonde 3 umfasst eine Sensoreinrichtung, wobei die Sensoreinrichtung Sensorelemente 11 umfasst. Die Sensorelemente 11 sind als Temperatursensoren ausgebildet und erfassen und/oder messen eine Temperatur, insbesondere in ihrer Umgebung. Die Sensorelemente 11 sind entlang einer Längserstreckung der Ablationssonde 3 angeordnet. Die Ablationssonde 3, insbesondere der Ablationsabschnitt 10 und/oder die Sensorelemente 11, sind in den Bilddatensätzen 6 bzw. den Bilddaten 7 sichtbar. Basierend auf einer Bildauswertung, im Speziellen basierend auf Algorithmen des maschinellen Lernens, werden die Ablationssonde 3, der Ablationsabschnitt 10 und die Sensorelemente 11 in den Bilddaten 7 detektiert und/oder segmentiert, wobei eine Sensorposition S(x,y,z) bestimmt wird. Die Sensorposition S(x,y,z) umfasst eine erste Sensorelementposition S_{E1}(x,y,z) und eine zweite Sensorelementposition S_{E2}(x,y,z). Beispielsweise bildet die Sensorposition S(x,y,z) ein Tupel S(x,y,z)={S_{E1}(x,y,z), S_{E2}(x,y,z)}. Die Sensoreinrichtung stellt eine zu einem Zeitpunkt t am einer Position X gemessene Temperatur Tₓ(t) bereit, zum Beispiel T_{E1}(t). Insbesondere kann die bereitgestellte Temperatur ein Tupel umfassend mehrere Temperaturen bilden, z.B. T(t)={ T_{E1}(t), T_{E2}(t)}.

Figur 3 zeigt beispielhaft einen Ausschnitt aus einer möglichen Bereitstellung der ermittelten bzw. bestimmten Temperaturverteilung T_{A}(x,y,z). Die Bereitstellung erfolgt beispielsweise auf einer Anzeige und/oder einem Monitor. Der Ausschnitt zeigt einen Abschnitt der Ablationssonde 3, insbesondere den Ablationsabschnitt 11. Der Ausschnitt kann insbesondere einen Ausschnitt aus einem Bild des Bilddatensatzes 6 umfassen und/oder zeigen. Als Temperaturverteilung T_{A}(x,y,z) wurden Bereiche um den Ablationsabschnitt 10 ermittelt, die eine gleiche Temperatur aufweisen und/oder in gleichen Temperaturintervallen 12 liegen. Die Temperaturverteilung wird vorzugsweise in den Bilddaten 7 angezeigt und/oder überlagert mit Bilddaten des Objektbereichs 8, sodass ein Benutzer und/oder behandelnde Person optisch Rückmeldung über die Temperatur im Ablationsbereich 5 und den Fortschritt der Ablationsbehandlung erhält.

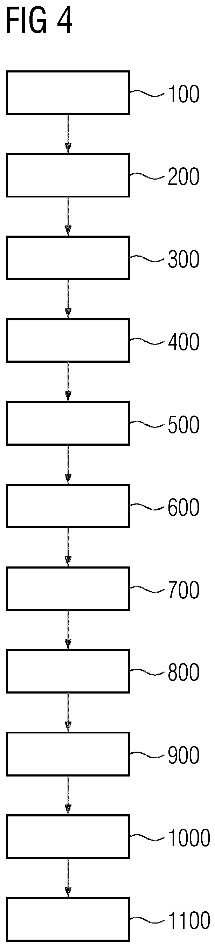

Figur 4 zeigt ein Flussdiagramm eines Ausführungsbeispiels eines Verfahrens zur Bestimmung einer Temperatur und/oder Temperaturverteilung in einem Ablationsbereich.

In einem Bereitstellungsschritt 100 wird ein erster Bilddatensatz 6 eines Objektbereichs 8 bereitgestellt. Das Bereitstellen erfolgt beispielsweise von einem Computertomographie-System 6 insbesondere über ein Interface. Der Bilddatensatz ist zu einem ersten Zeitpunkt t1 aufgenommen und umfasst eine Mehrzahl an Bilddaten 7, zum Beispiel Bilddaten in Form von medizinischen Bildern und/oder CT-Aufnahmen. Die medizinischen Bilddaten 7 bilden Bilddaten spektraler Computertomographie und/oder multi energy CT-Aufnahmen. Die Bilddaten 7 zeigen den Ablationsbereich 5 und die Ablationssonde 3.

In einem Bestimmungsschritt 200 wird basierend auf dem ersten Bilddatensatz 6, insbesondere den Bilddaten 7, eine erste Sensorposition S(x,y,z) bestimmt. Die Sensorposition S(x,y,z) umfasst und/oder bildet die Position, an der eine erste Temperatur ermittelt wird und/oder wurde. Die Sensorposition S(x,y,z) wird beispielsweise basierend auf einer Bildauswertung und/oder Bildanalyse der Bilddaten 7 bestimmt, beispielsweise durch Detektion der Sensorelemente 11 in den Bilddaten 7.

In einem ersten Ermittlungsschritt 300 werden die im Schritt 100 bereitgestellten Bilddaten 7 analysiert und/oder ausgewertet. Basierend auf den Bilddaten 7 wird für den Objektbereiche eine Dichteverteilung und/oder Schwächungswertverteilung bestimmt. Beispielsweise werden Bereiche gleiche Dichte und/oder Schwächungswerte bestimmt, segmentiert und/oder geclustert. Die Dichte kann basierend auf den Bilddaten 7 bestimmt werden, da Bereiche gleicher Dichte und/oder gleichen Gewebes einen gleichen und/oder ähnlichen Schwächungswert aufweisen. Insbesondere wird die Dichte, Dichteverteilung, der Schwächungswert und/oder Schwächungswertverteilung für Bereiche um die Sensorelemente 11 ermittelt.

In einem Bereitstellungsschritt 400 wird eine erste Temperatur bereitgestellt. Die erste Temperatur ist eine Temperatur gemessen von mindestens einem der Sensorelemente 11. Die erste Temperatur ist insbesondere gemessen zum Zeitpunkt t1. Die erste Temperatur beschreibt mit anderen Worten die Temperatur zum Zeitpunkt der Aufnahme des ersten Bilddatensatzes 6 an der ersten Sensorposition S(x,y,z).

In einem Bereitstellungsschritt 500 wird ein zweiter Bilddatensatz 6 eines Objektbereichs 8 bereitgestellt. Das Bereitstellen erfolgt beispielsweise von einem Computertomographie-System 6 insbesondere über ein Interface. Der Bilddatensatz ist zu einem ersten Zeitpunkt t2 aufgenommen und umfasst eine Mehrzahl an Bilddaten 7, zum Beispiel Bilddaten in Form von medizinischen Bildern und/oder CT-Aufnahmen. Die medizinischen Bilddaten 7 bilden Bilddaten spektraler Computertomographie und/oder multi energy CT-Aufnahmen. Die Bilddaten 7 zeigen den Ablationsbereich 5 und die Ablationssonde 3.

In einem Bestimmungsschritt 600 wird basierend auf dem zweiten Bilddatensatz 6, insbesondere den Bilddaten 7, eine zweite Sensorposition S'(x,y,z) bestimmt. Die Sensorposition S' (x,y,z) umfasst und/oder bildet die Position, an der eine zweite Temperatur ermittelt wird und/oder wurde. Die Sensorposition S'(x,y,z) wird beispielsweise basierend auf einer Bildauswertung und/oder Bildanalyse der Bilddaten 7 bestimmt, beispielsweise durch Detektion der Sensorelemente 11 in den Bilddaten 7.

In einem zweiten Ermittlungsschritt 700 werden die im Schritt 500 bereitgestellten Bilddaten 7 analysiert und/oder ausgewertet. Basierend auf den Bilddaten 7 wird für den Objektbereiche eine Dichteverteilung und/oder Schwächungswertverteilung bestimmt. Insbesondere wird die Dichte, Dichteverteilung, der Schwächungswert und/oder Schwächungswertverteilung für Bereiche um die Sensorelemente 11 ermittelt.

In einem Bereitstellungsschritt 800 wird eine zweite Temperatur bereitgestellt. Die zweite Temperatur ist eine Temperatur gemessen von mindestens einem der Sensorelemente 11. Die zweite Temperatur ist insbesondere gemessen zum Zeitpunkt t2. Die zweite Temperatur beschreibt mit anderen Worten die Temperatur zum Zeitpunkt der Aufnahme des zweiten Bilddatensatzes 6 an der zweiten Sensorposition S'(x,y,z).

In einem Bestimmungsschritt 900 wird für den Ablationsbereich eine Dichte, Dichteverteilung, Schwächungswert und/oder Schwächungswertverteilung bestimmt, wobei das Bestimmen basierend auf den Bilddaten des ersten Bilddatensatzes, des zweiten Bilddatensatzes und/oder weiterer bereitgestellter Bilddatensätze 7 des Objektbereichs 8. Die weiteren Bilddatensätze 7 sind aufgenommen und/oder bereitgestellt wie der erste Bilddatensatz 7.

In einem Bestimmungsschritt 1000 eine Temperatur und/oder eine Temperaturverteilung T_{A}(x,y,z) im Ablationsbereich 5 bestimmt. Dies kann basierend auf einer Kalibrationsfunktion erfolgen. Die Kalibrationsfunktion wird beispielsweise basierend auf der ersten Temperatur und der zugehörigen Dichte, Dichteverteilung, Schwächungswert und/oder Schwächungswertverteilung an der Sensorposition S(x,y,z) sowie der zweiten Temperatur und der zugehörigen basierend auf den zweiten Bilddaten ermittelten Dichte, Dichteverteilung, Schwächungswert und/oder Schwächungswertverteilung an der zweiten Sensorposition S'(x,y,z). Die Kalibrationsfunktion ist ausgebildet einer Dichte, Dichteverteilung, Schwächungswert und/oder Schwächungswertverteilung eine Temperatur zuzuordnen. Die Temperatur und/oder Temperaturverteilung T_{A}(x,y,z) wird beispielsweise durch Anwenden der Kalibrationsfunktion auf die Dichte, Dichteverteilung, Schwächungswert und/oder Schwächungswertverteilung im Ablationsbereich 5 erhalten.

In einem Bereitstellungsschritt 1100 wird die bestimmte Temperatur und/oder Temperaturverteilung T_{A}(x,y,z) im Ablationsbereich 5 bereitgestellt, beispielswiese optisch auf einer Anzeige angezeigt. Insbesondere wird die Temperatur und/oder Temperaturverteilung T_{A}(x,y,z) in den Bilddaten 7 und/oder Bilddatensatz eingezeichnet und/oder angezeigt. Ein Benutzer kann so den Fortschritt der Ablationsbehandlung auf der Anzeige verfolgen.

## Patentansprüche

1. Computerprogrammprodukt umfassend Programmcodemittel um eine Ablationsanordnung (1) nach Anspruch 12 dazu zu veranlassen ein Verfahren zur Bestimmung einer Temperatur und/oder einer Temperaturverteilung (T_{A}) in einem Ablationsbereich (5) auszuführen, wobei das Verfahren folgende Schritte umfasst:
- Bereitstellen (100) eines ersten Bilddatensatzes (6) eines Objektbereichs (8), wobei der erste Bilddatensatz (6) bei unterschiedlicher spektraler Verteilung einer Röntgenstrahlung aufgenommene medizinische Bilddaten (7) umfasst, wobei der Objektbereich (8) den Ablationsbereich (5) umfasst,
- Bestimmen (200) einer ersten Sensorposition (S) im Objektbereich (8) basierend auf dem ersten Bilddatensatz (6),
- Ermitteln (300) einer ersten Dichte, einer ersten Dichteverteilung, eines ersten Schwächungswertes, einer ersten Schwächungswertverteilung und/oder einer ersten Gewebeverteilung in einem Umgebungsbereich der ersten Sensorposition (S) basierend auf dem ersten Bilddatensatz (6),
- Bereitstellen (400) einer ersten Temperatur für die erste Sensorposition (S),
- Bereitstellen (500) eines zweiten Bilddatensatzes (6) des Objektbereichs (8), wobei der zweite Bilddatensatz (6) bei unterschiedlicher spektraler Verteilung einer Röntgenstrahlung aufgenommene medizinische Bilddaten (7) umfasst,
- Bestimmen (600) einer zweiten Sensorposition (S') im Objektbereich (8) basierend auf dem zweiten Bilddatensatz (6),
- Ermitteln (700) einer zweiten Dichte, einer zweiten Dichteverteilung, eines zweiten Schwächungswerts, einer zweiten Schwächungswertverteilung und/oder einer zweiten Gewebeverteilung in einem Umgebungsbereich der zweiten Sensorposition (S') basierend auf dem zweiten Bilddatensatz (6),
- Bereitstellen (700) einer zweiten Temperatur für die zweite Sensorposition (S'),
- Bestimmen (1000) einer Temperatur und/oder einer Temperaturverteilung (T_{A}) im Ablationsbereich (5) basierend auf der ersten Temperatur, der ersten Sensorposition (S), der zweiten Temperatur, der zweiten Sensorposition (S'), der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung,
- Bereitstellen (1100) der bestimmten Temperatur und/oder Temperaturverteilung (T_{A}),
wobei die erste Temperatur und/oder die zweite Temperatur von einer Sensoreinrichtung bereitgestellt wird, wobei die Sensoreinrichtung ein erstes Sensorelement (11) und ein zweites Sensorelement (11) umfasst, wobei das erste Sensorelement (11) und das zweite Sensorelement (11) in einem Sensorabstand beabstandet sind, wobei die erste Sensorposition (S) eine erste Sensorelementposition (S_{E1}) für das erste Sensorelement (11) und eine zweite Sensorelementposition (S_{E2}) für das zweite Sensorelement (11) umfasst, wobei die erste Temperatur und/oder die zweite Temperatur einen ersten Temperaturwert für das erste Sensorelement (11) und einen zweiten Temperaturwert für das zweite Sensorelement (11) umfasst.

2. Computerprogrammprodukt nach Anspruch 1, **dadurch gekennzeichnet, dass** im Schritt Ermitteln (700) einer zweiten Dichteverteilung, einer zweiten Schwächungswertverteilung und/oder einer zweiten Gewebe-verteilung die zweiten Dichteverteilung, die zweiten Schwächungswertverteilung und/oder die zweiten Gewebeverteilung im Objektbereich (8) ermittelt wird.

3. Computerprogrammprodukt nach Anspruch 1 oder 2, **dadurch gekennzeichnet, dass** im Schritt Ermitteln (300) einer ersten Dichte, einer ersten Dichteverteilung, eines ersten Schwächungswerts, einer ersten Schwächungswertverteilung und/oder einer erste Gewebeverteilung die erste Dichte, die erste Dichteverteilung, der erste Schwächungswert, die erste Schwächungswertverteilung und/oder die erste Gewebeverteilung in dem Objektbereich (8) ermittelt wird.

4. Computerprogrammprodukt nach einem der vorherigen Ansprüche, **gekennzeichnet durch**:
- Bestimmen einer Dichteveränderung, einer Schwächungswertveränderung und/oder einer Gewebeverteilungsänderung basierend auf der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung, wobei die Temperatur und/oder Temperaturverteilung (T_{A}) im Ablationsbereich (5) basierend auf der ersten Temperatur, der zweiten Temperatur, der Dichteveränderung, der Schwächungswertveränderung und/oder der Gewebeverteilungsveränderung bestimmt wird.

5. Computerprogrammprodukt nach einem der vorherigen Ansprüche, **gekennzeichnet durch**:
- Bestimmen einer Kalibrationsfunktion basierend auf der ersten Temperatur, der ersten Sensorposition (S), der zweiten Temperatur, der zweiten Sensorposition (S'), der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung, wobei die Kalibrationsfunktion einer Dichte, einem Schwächungswert und/oder einem Gewebe eine Temperatur zuordnet.

6. Computerprogrammprodukt nach einem der vorherigen Ansprüche, **dadurch gekennzeichnet, dass** das Bereitstellen (100, 500) eines ersten und/oder eines zweiten Bilddatensatzes (7) eines Objektbereichs (8) ein Artefaktbereinigung umfasst, wobei die Artefaktbereinigung Bildartefakte und/oder Metallartefakte in den medzinischen Bilddaten (7) bereinigt und/oder verringert.

7. Computerprogrammprodukt nach einem der vorherigen Ansprüche, **dadurch gekennzeichnet, dass** die erste Temperatur und/oder die zweite Temperatur von einer Sensoreinrichtung bereitgestellt wird, wobei die Sensoreinrichtung von einer Ablationsvorrichtung (2) umfasst ist, wobei die Ablationsvorrichtung einen Ablationsabschnitt (10) zur thermischen Ablation umfasst.

8. Computerprogrammprodukt nach einem der vorherigen Ansprüche, **gekennzeichnet durch**:
- Bereitstellen eines dritten Bilddatensatzes des Objektbereichs, wobei der dritte Bilddatensatz (6) bei unterschiedlicher spektraler Verteilung einer Röntgenstrahlung aufgenommene medizinische Bilddaten (7)umfasst,
- Bestimmen einer dritten Temperatur und/oder einer dritten Temperaturverteilung im Ablationsbereich (5) basierend auf der ersten Temperatur, der ersten Sensorposition (S), der zweiten Temperatur, der zweiten Sensorposition (S'), der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung,
- Ermitteln eines geplanten Temperaturverlaufs im Ablationsbereich (5) basierend auf der ersten, zweiten und dritten Temperatur und/oder Temperaturverteilung (T_{A}),
- Bereitstellen des geplanten Temperaturverlaufs.

9. Computerprogrammprodukt nach Anspruch 8, **dadurch gekennzeichnet, dass** Ablationsbetriebsdaten und/oder Gewebeparameter bereitgestellt werden, wobei der geplante Temperaturverlauf basierend auf den Ablationsbetriebsdaten und/oder Gewebeparameter ermittelt wird.

10. Computerprogrammprodukt nach einem der Ansprüche 8 oder 9, **dadurch gekennzeichnet, dass** basierend auf dem geplanten Temperaturverlauf eine geplante Restablationsdauer ermittelt wird, wobei der geplante Restablationsdauer bereitgestellt wird.

11. Computerprogrammprodukt nach einem der vorherigen Ansprüche, **dadurch gekennzeichnet, dass** basierend auf der ersten Temperatur, der zweiten Temperatur, der dritten Temperatur, der ersten Temperaturverteilung, der zweiten Temperaturverteilung und/oder der dritten Temperaturverteilung Wärmeverlustbereiche im Ablationsbereich (5) ermittelt werden, wobei die ermittelten Wärmeverlustbereich bereitgestellt werden.

12. Ablationsanordnung (1) umfassend eine Ablationsvorrichtung (2) und ein System (1) zur Bestimmung einer Temperatur und/oder einer Temperaturverteilung (T_{A}) in einem Ablationsbereich (5), wobei die Ablationsvorrichtung (2) eine Sensoreinrichtung zur Bereitstellung der ersten, zweiten und/oder dritten Temperatur und/oder Temperaturverteilung aufweist, wobei das System (1) ein Bilddatenbereitstellungsmodul (20), ein Bilddatenauswertemodul (21), ein Sensordatenbereitstellungsmodul (22), ein Positionsbestimmungsmodul (23), ein Temperaturbestimmungsmodul (24) und ein Bereitstellungsmodul (25) umfasst, wobei:
- Das Bilddatenbereitstellungsmodul (20) ausgebildet ist, einen ersten Bilddatensatzes (6) eines Objektbereichs (8) bereitzustellen, wobei der erste Bilddatensatz (6) bei unterschiedlicher spektraler Verteilung einer Röntgenstrahlung aufgenommene medizinische Bilddaten (7) umfasst, wobei der Objektbereich (8) den Ablationsbereich (5) umfasst,
- Das Bilddatenauswertemodul (21) ausgebildet ist eine erste Dichte, eine erste Dichteverteilung, einen ersten Schwächungswert, eine erste Schwächungswertverteilung und/oder eine erste Gewebeverteilung in mindestens einem Abschnitt des Objektbereichs basierend auf dem ersten Bilddatensatz (6) zu ermitteln,
- Das Positionsbestimmungsmodul (23) ausgebildet ist, eine erste Sensorposition (S) im Objektbereich (8) basierend auf dem ersten Bilddatensatz (6) zu bestimmen,
- Das Sensordatenbereitstellungsmodul (22) ausgebildet ist, eine erste Temperatur für die erste Sensorposition bereitzustellen (S),
- Das Bilddatenbereitstellungsmodul (20) ausgebildet ist, einen zweiten Bilddatensatz (6) des Objektbereichs (8) bereitzustellen, wobei der zweite Bilddatensatz (6) bei unterschiedlicher spektraler Verteilung einer Röntgenstrahlung aufgenommene medizinische Bilddaten (7) umfasst,
- Das Positionsbestimmungsmodul (23) ausgebildet ist, eine zweite Sensorposition (S') im Objektbereich (8) basierend auf dem zweiten Bilddatensatz (6) zu bestimmen,
- Das Sensordatenbereitstellungsmodul (22) ausgebildet ist, eine zweite Temperatur für die zweite Sensorposition (S') bereitzustellen,
- Das Bilddatenauswertemodul (21) ausgebildet ist, eine zweite Dichte, eine zweite Dichteverteilung, einen zweiten Schwächungswert, eine zweite Schwächungswertverteilung und/oder eine zweite Gewebeverteilung in einem Umgebungsbereich der zweiten Sensorposition (S') basierend auf dem zweiten Bilddatensatz (6) zu ermitteln,
- Das Temperaturbestimmungsmodul (24) ausgebildet ist, eine Temperatur und/oder eine Temperaturverteilung (T_{A}) im Ablationsbereich (5) basierend auf der ersten Temperatur, der ersten Sensorposition (S), der zweiten Temperatur, der zweiten Sensorposition (S'), der ersten Dichte, der ersten Dichteverteilung, dem ersten Schwächungswert, der ersten Schwächungswertverteilung, der ersten Gewebeverteilung, der zweiten Dichte, der zweiten Dichteverteilung, dem zweiten Schwächungswert, der zweiten Schwächungswertverteilung und/oder der zweiten Gewebeverteilung zu bestimmen,
- Das Bereitstellungsmodul (25) ausgebildet ist, die bestimmte Temperatur und/oder Temperaturverteilung (T_{A}) bereitzustellen
- Die Sensoreinrichtung ausgebildet ist die erste Temperatur und/oder die zweite Temperatur bereitzustellen, wobei die Sensoreinrichtung ein erstes Sensorelement (11) und ein zweites Sensorelement (11) umfasst, wobei das erste Sensorelement (11) und das zweite Sensorelement (11) in einem Sensorabstand beabstandet sind, wobei die erste Sensorposition (S) eine erste Sensorelementposition (S_{E1}) für das erste Sensorelement (11) und eine zweite Sensorelementposition (S_{E2}) für das zweite Sensorelement (11) umfasst, wobei die erste Temperatur und/oder die zweite Temperatur einen ersten Temperaturwert für das erste Sensorelement (11) und einen zweiten Temperaturwert für das zweite Sensorelement (11) umfasst.

## Claims

1. Computer program product comprising program code means for causing an ablation assembly (1) according to claim 12 to perform a method for ascertaining a temperature and/or a temperature distribution (T_{A}) in an ablation region (5), wherein the method comprises the following steps:
- providing (100) a first image dataset (6) of an object region (8), wherein the first image dataset (6) comprises medical image data (7) recorded with varying spectral distribution of an X-ray, wherein the object region (8) comprises the ablation region (5),
- ascertaining (200), based on the first image dataset (6), a first sensor position (S) in the object region (8),
- determining (300), based on the first image dataset (6), a first density, a first density distribution, a first attenuation value, a first attenuation value distribution and/or a first tissue distribution in a region surrounding the first sensor position (S),
- providing (400) a first temperature for the first sensor position (S),
- providing (500) a second image dataset (6) of the object region (8), wherein the second image dataset (6) comprises medical image data (7) recorded with varying spectral distribution of an X-ray,
- ascertaining (600), based on the second image dataset (6), a second sensor position (S') in the object region (8),
- determining (700), based on the second image dataset (6), a second density, a second density distribution, a second attenuation value, a second attenuation value distribution and/or a second tissue distribution in a region surrounding the second sensor position (S'),
- providing (700) a second temperature for the second sensor position (S'),
- ascertaining (1000) a temperature and/or a temperature distribution (T_{A}) in the ablation region (5) based on the first temperature, the first sensor position (S), the second temperature, the second sensor position (S'), the first density, the first density distribution, the first attenuation value, the first attenuation value distribution, the first tissue distribution, the second density, the second density distribution, the second attenuation value, the second attenuation value distribution and/or the second tissue distribution,
- providing (1100) the ascertained temperature and/or temperature distribution (T_{A}),
wherein the first temperature and/or the second temperature is provided by a sensor facility, wherein the sensor facility comprises a first sensor element (11) and a second sensor element (11), wherein the first sensor element (11) and the second sensor element (11) are spaced apart at a sensor distance, wherein the first sensor position (S) comprises a first sensor element position (S_{E1}) for the first sensor element (11) and a second sensor element position (S_{E2}) for the second sensor element (11), wherein the first temperature and/or the second temperature comprises a first temperature value for the first sensor element (11) and a second temperature value for the second sensor element (11).

2. Computer program product according to claim 1, **characterised in that** in the step of determining (700) a second density distribution, a second attenuation value distribution and/or a second tissue distribution, the second density distribution, the second attenuation value distribution and/or the second tissue distribution are determined in the object region (8).

3. Computer program product according to claim 1 or 2, **characterised in that** in the step of determining (300) a first density, a first density distribution, a first attenuation value, a first attenuation value distribution and/or a first tissue distribution, the first density, the first density distribution, the first attenuation value, the first attenuation value distribution and/or the first tissue distribution are determined in the object region (8).

4. Computer program product according to one of the preceding claims, **characterised by**:
- ascertaining a change in density, a change in attenuation value and/or a change in tissue distribution based on the first density, the first density distribution, the first attenuation value, the first attenuation value distribution, the first tissue distribution, the second attenuation value, the second attenuation value distribution and/or the second tissue distribution, wherein the temperature and/or the temperature distribution (T_{A}) in the ablation region (5) is ascertained based on the first temperature, the second temperature, the change in density, the change in attenuation value and/or the change in tissue distribution.

5. Computer program product according to one of the preceding claims, **characterised by**
- ascertaining a calibration function based on the first temperature, the first sensor position (S), the second temperature, the second sensor position (S'), the first density, the first density distribution, the first attenuation value, the first attenuation value distribution, the first tissue distribution, the second density, the second density distribution, the second attenuation value, the second attenuation value distribution and/or the second tissue distribution, wherein the calibration function assigns a temperature to a density, an attenuation value and/or a tissue.

6. Computer program product according to one of the preceding claims, **characterised in that** providing (100, 500) a first and/or a second image dataset (7) of an object region (8) comprises artifact correction, wherein the artifact correction corrects and/or reduces image artifacts and/or metal artifacts in the medical image data (7).

7. Computer program product according to one of the preceding claims, **characterised in that** the first temperature and/or the second temperature is/are provided by a sensor facility, wherein the sensor facility is comprised by an ablation apparatus (2), wherein the ablation apparatus comprises an ablation section (10) for thermal ablation.

8. Computer program product according to one of the preceding claims, **characterised by**:
- providing a third image dataset of the object region, wherein the third image dataset (6) comprises medical image data (7) recorded with varying spectral distribution of an X-ray,
- ascertaining a third temperature and/or a third temperature distribution in the ablation region (5) based on the first temperature, the first sensor position (S), the second temperature, the second sensor position (S'), the first density, the first density distribution, the first attenuation value, the first attenuation value distribution, the first tissue distribution, the second density, the second density distribution, the second attenuation value, the second attenuation value distribution and/or the second tissue distribution,
- determining a planned temperature curve in the ablation region (5) based on the first, second and third temperature and/or temperature distribution (T_{A}),
- providing the planned temperature curve.

9. Computer program product according to claim 8, **characterised in that** the ablation operating data and/or the tissue parameters are provided, wherein the planned temperature curve is determined based on the ablation operating data and/or the tissue parameters.

10. Computer program product according to claim 8 or 9, **characterised in that** a planned residual ablation duration is determined based on the planned temperature curve, wherein the planned residual ablation duration is provided.

11. Computer program product according to one of the preceding claims, **characterised in that** heat loss regions in the ablation region (5) are determined based on the first temperature, the second temperature, the third temperature, the first temperature distribution, the second temperature distribution and/or the third temperature distribution, wherein the determined heat loss regions are provided.

12. Ablation assembly (1) comprising an ablation apparatus (2) and a system (1) for ascertaining a temperature and/or a temperature distribution (T_{A}) in an ablation region (5),
wherein the ablation apparatus (2) has a sensor facility for providing the first, second and/or third temperature and/or temperature distribution,
wherein the system (1) comprises an image data provision module (20), an image data evaluation module (21), a sensor data provision module (22), a position ascertainment module (23), a temperature ascertainment module (24) and a provision module (25), wherein:
- the image data provision module (20) is designed to provide a first image dataset (6) of an object region (8), wherein the first image dataset (6) comprises medical image data (7) recorded with varying spectral distribution of an X-ray, wherein the object region (8) comprises the ablation region (5),
- the image evaluation module (21) is designed to determine, based on the first image dataset (6), a first density, a first density distribution, a first attenuation value, a first attenuation value distribution and/or a first tissue distribution in at least one section of the object region,
- the position ascertainment module (23) is designed to ascertain, based on the first image dataset (6), a first sensor position (S) in the object region (8),
- the sensor data provision module (22) is designed to provide (S) a first temperature for the first sensor position,
- the image data provision module (20) is designed to provide a second image dataset (6) of the object region (8), wherein the second image dataset (6) comprises medical image data (7) recorded with varying spectral distribution of an X-ray,
- the position ascertainment module (23) is designed to ascertain, based on the second image dataset (6), a second sensor position (S') in the object region (8),
- the sensor data provision module (22) is designed to provide a second temperature for the second sensor position (S'),
- the image evaluation module (21) is designed to ascertain, based on the second image dataset (6), a second density, a second density distribution, a second attenuation value, a second attenuation value distribution and/or a second tissue distribution in a region surrounding the second sensor position (S'),
- the temperature ascertainment module (24) is designed to ascertain a temperature and/or a temperature distribution (T_{A}) in the ablation region (5) based on the first temperature, the first sensor position (S), the second temperature, the second sensor position (S'), the first density, the first density distribution, the first attenuation value, the first attenuation value distribution, the first tissue distribution, the second density, the second density distribution, the second attenuation value, the second attenuation distribution value and/or the second tissue distribution,
- the provision module (25) is designed to provide the ascertained temperature and/or temperature distribution (T_{A}),
- the sensor facility is designed to provide the first temperature and/or the second temperature, wherein the sensor facility comprises a first sensor element (11) and a second sensor element (11), wherein the first sensor element (11) and the second sensor element (11) are spaced apart at a sensor distance, wherein the first sensor position (S) comprises a first sensor element position (S_{E1}) for the first sensor element (11) and a second sensor element position (S_{E2}) for the second sensor element (11), wherein the first temperature and/or the second temperature comprises a first temperature value for the first sensor element (11) and a second temperature value for the second sensor element (11).

## Revendications

1. Produit de programme d'ordinateur comprenant un moyen de code de programme pour faire qu'un agencement (1) d'ablation suivant la revendication 12 exécute un procédé de détermination d'une température et/ou d'une répartition (T_{A}) de température dans une zone (5) d'ablation, dans lequel le procédé comprend les stades suivants :
- se procurer (100) un premier ensemble (6) de données d'image d'une zone (8) d'un objet, dans lequel le premier ensemble (6) de données d'image comprend des données (7) d'image médicale enregistrées à une répartition spectrale différente d'un rayonnement X, dans lequel la zone (8) de l'objet comprend la zone (5) d'ablation,
- déterminer (200) une première position (S) de capteur dans la zone (8) de l'objet sur la base du premier ensemble (6) de données d'image,
- déterminer (300) une première densité, une première répartition de densité, une première valeur d'atténuation, une première répartition de valeurs d'atténuation et/ou une première répartition de tissu dans une zone environnante de la première position (S) du capteur sur la base du premier ensemble (6) de données d'image,
- se procurer (400) une première température pour la première position (S) du capteur,
- se procurer (500) un deuxième ensemble (6) de données d'image de la zone (8) de l'objet, dans lequel le deuxième ensemble (6) de données d'image comprend des données (7) d'image médicale enregistrées à une répartition spectrale différente d'un rayonnement X,
- déterminer (600) une deuxième position (S') de capteur dans la zone (8) de l'objet sur la base du deuxième ensemble (6) de données d'image,
- déterminer (700) une deuxième densité, une deuxième répartition de densité, une deuxième valeur d'atténuation, une deuxième répartition de valeurs d'atténuation et/ou une deuxième répartition de tissu dans une zone environnante de la deuxième position (S') de capteur sur la base du deuxième ensemble (6) de données d'image,
- se procurer (700) une deuxième température pour la deuxième position (S') du capteur,
- déterminer (1000) une température et/ou une répartition (T_{A}) de température dans la zone (5) d'ablation sur la base de la première température, de la première position (S) du capteur, de la deuxième température, de la deuxième position (S') du capteur, de la première densité, de la première répartition de densité, de la première valeur d'atténuation, de la première répartition de valeurs d'atténuation, de la première répartition de tissu, de la deuxième densité, de la deuxième répartition de densité, de la deuxième valeur d'atténuation, de la deuxième répartition de valeurs d'atténuation et/ou de la deuxième répartition de tissu,
- se procurer (1100) la température déterminée et/ou la répartition (T_{A}) de température,
dans lequel on se procure la première température et/ou la deuxième température par un dispositif capteur, dans lequel le dispositif capteur comprend un premier élément (11) et un deuxième élément (11) capteur, dans lequel le premier élément (11) capteur et le deuxième élément (11) capteur sont à distance à une distance de capteur, dans lequel la première position (S) de capteur comprend une première position (S_{E1}) de l'élément de capteur pour le premier élément (11) de capteur et une deuxième position (S_{E2}) d'élément de capteur pour le deuxième élément (11) de capteur, dans lequel la première température et/ou la deuxième température comprend une première valeur de température pour le premier élément (11) de capteur et une deuxième valeur de température pour le deuxième élément (11) de capteur.

2. Produit de programme d'ordinateur suivant la revendication 1, **caractérisé en ce que**, dans le stade détermination (700) d'une deuxième répartition de densité, d'une deuxième répartition de valeurs d'atténuation et/ou d'une deuxième répartition de tissu, on détermine la deuxième répartition de densité, la deuxième répartition de valeurs d'atténuation et/ou la deuxième répartition de tissu dans la partie (8) de l'objet.

3. Produit de programme d'ordinateur suivant la revendication 1 ou 2, **caractérisé en ce que**, dans le stade de détermination (300) d'une première densité, d'une première répartition de densité, d'une première valeur d'atténuation, d'une répartition de valeurs d'atténuation et/ou d'une première répartition de tissu, on détermine la première densité, la première répartition de densité, la première valeur d'atténuation, la première répartition de valeurs d'atténuation et/ou la première répartition de tissu dans la zone (8) de l'objet.

4. Produit de programme d'ordinateur suivant l'une des revendications précédentes, **caractérisé par** :
- détermination d'une variation de densité, d'une variation de valeurs d'atténuation et/ou d'une variation de répartition de tissu, sur la base de la première densité, de la première répartition de densité, de la première valeur d'atténuation, de la première répartition de valeurs d'atténuation, de la première répartition de tissu, de la deuxième densité, de la deuxième répartition de densité, de la deuxième valeur d'atténuation, de la deuxième répartition de valeurs d'atténuation et/ou de la deuxième répartition de tissu, dans lequel on détermine la température et/ou la répartition (T_{A}) de température dans la zone (5) d'ablation, sur la base de la première température, de la deuxième température, de la variation de densités, de la variation de valeurs d'atténuation et/ou de la variation de la répartition de tissu.

5. Produit de programme d'ordinateur suivant l'une des revendications précédentes, **caractérisé par** :
- détermination d'une fonction d'étalonnage sur la base de la première température, de la première position (S) du capteur, de la deuxième température, de la deuxième position (S') du capteur, de la première densité, de la première répartition de densité, de la première valeur d'atténuation, de la première répartition de valeurs d'atténuation, de la première répartition de tissu, de la deuxième densité et de la deuxième répartition de densité, de la deuxième valeur d'atténuation, de la deuxième répartition de valeurs d'atténuation et/ou d'une deuxième répartition de tissu, dans lequel la fonction d'étalonnage affecte une température à une densité, à une valeur d'atténuation et/ou à un tissu.

6. Produit de programme d'ordinateur suivant l'une des revendications précédentes, **caractérisé en ce que** se procurer (100, 500) un premier et/ou un deuxième ensembles (7) de données d'image d'une zone (8) de l'objet comprend une épuration d'artefacts, dans lequel l'épuration d'artefacts épure et/ou diminue des artefacts d'image et/ou des artefacts de métal dans les données (7) d'image médicale.

7. Produit de programme d'ordinateur suivant l'une des revendications précédentes, **caractérisé en ce que** l'on se procure la première température et/ou la deuxième température par un dispositif capteur, dans lequel le dispositif capteur est compris par une installation (2) d'ablation, dans lequel l'installation d'ablation comprend une partie (10) d'ablation pour l'ablation thermique.

8. Produit de programme d'ordinateur suivant l'une des revendications précédentes, **caractérisé par** :
- se procurer un troisième ensemble de données d'image de la zone de l'objet, dans lequel le troisième ensemble (6) de données d'image comprend des données (7) d'image médicale enregistrées à une répartition spectrale différente d'un rayonnement X,
- déterminer une troisième température et/ou une troisième répartition de température dans la zone (5) d'ablation sur la base de la première température, de la première position (S) du capteur, de la deuxième température, de la deuxième position (S') du capteur, de la première densité, de la première répartition de densité, de la première valeur d'atténuation, de la première répartition de valeurs d'atténuation, de la première répartition de tissu, de la deuxième densité, de la deuxième répartition de densité, de la deuxième valeur d'atténuation, de la deuxième répartition de valeurs d'atténuation et/ou de la deuxième répartition de tissu,
- déterminer une courbe de température planifiée dans la zone (5) d'ablation sur la base de la première, deuxième et troisième températures et/ou de la répartition (T_{A}) de température,
- mettre à disposition la courbe de température planifiée.

9. Produit de programme d'ordinateur suivant la revendication 8, **caractérisé en ce que** l'on se procure les données de fonctionnement d'ablation et/ou des paramètres de tissu, dans lequel on détermine la courbe de température planifiée sur la base des données de fonctionnement d'ablation et/ou des paramètres de tissu.

10. Produit de programme d'ordinateur suivant l'une des revendications 8 ou 9, **caractérisé en ce que** l'on détermine une durée d'ablation restante planifiée sur la base de la courbe de température planifiée, dans lequel on se procure la durée d'ablation restante planifiée.

11. Produit de programme d'ordinateur suivant l'une des revendications précédentes, **caractérisé en ce que**, sur la base de la première température, de la deuxième température, de la troisième température, de la première répartition de température, de la deuxième répartition de température et/ou de la troisième répartition de température, on détermine des zones de perte de chaleur dans la zone (5) d'ablation, dans lequel on se procure la zone de perte de chaleur déterminée.

12. Agencement (1) d'ablation comprenant une installation (2) d'ablation et un système (1) de détermination de la température et/ou d'une répartition (T_{A}) de la température dans une zone (5) d'ablation, dans lequel l'installation (2) d'ablation a un dispositif capteur pour disposer de la première, deuxième et/ou troisième températures et/ou d'une répartition de températures, dans lequel le système (1) comprend un module (20) de mise à disposition de données d'image, un module (21) d'évaluation de données d'image, un module (22) de mise à disposition de données de capteur, un module (23) de détermination de la position, un module (24) de détermination de la température et un module (25) de mise à disposition, dans lequel :
- le module (20) de mise à disposition de données d'image est constitué pour mettre à disposition un premier ensemble (6) de données d'image d'une zone (8) d'un objet, dans lequel le premier ensemble (6) de données d'image comprend des données (7) d'image médicales enregistrées à une répartition spectrale différente d'un rayonnement X, dans lequel la zone (8) de l'objet comprend la zone (5) d'ablation,
- le module (21) d'évaluation de données d'image est constitué pour déterminer une première densité, une première répartition de densité, une première valeur d'atténuation, une première répartition de valeurs d'atténuation et/ou une première répartition de tissu dans au moins une partie de la zone de l'objet sur la base du premier ensemble (6) de données d'image,
- le module (23) de détermination de la position est constitué pour déterminer une première position (S) du capteur dans la zone (8) de l'objet, sur la base du premier ensemble (6) de données d'image,
- le module (22) de mise à disposition de données de capteur est constitué pour mettre (S) à disposition une première température pour la première position du capteur,
- le module (20) de mise à disposition de données d'image est constitué pour mettre à disposition le deuxième ensemble (6) de données d'image de la zone (8) de l'objet, dans lequel le deuxième ensemble (6) de données d'image comprend des données (7) d'image médicale enregistrées à une répartition spectrale différente d'un rayonnement X,
- le module (23) de détermination de la position est constitué pour déterminer une deuxième position (S') du capteur dans la zone (8) de l'objet sur la base du deuxième ensemble (6) de données d'image,
- le module (22) de mise à disposition de données du capteur est constitué pour mettre à disposition une deuxième température pour la deuxième position (S') du capteur,
- le module (21) d'évaluation de données d'image est constitué pour déterminer une deuxième densité, une deuxième répartition de densité, une deuxième valeur d'atténuation, une deuxième répartition de valeurs d'atténuation et/ou une deuxième répartition de tissu dans une zone environnante de la deuxième position (S') de capteur sur la base du deuxième ensemble (6) de données d'image,
- le module (24) de détermination de la température est constitué pour déterminer une température et/ou une répartition (T_{A}) de température dans la zone (5) d'ablation sur la base de la première température, de la première position (S) du capteur, de la deuxième température, de la deuxième position (S') du capteur, de la première densité, de la première répartition de densité, de la première valeur d'atténuation, de la première répartition de valeurs d'atténuation, de la première répartition de tissu, de la deuxième densité, de la deuxième répartition de densité, de la deuxième valeur d'atténuation, de la deuxième répartition de valeurs d'atténuation et/ou de la deuxième répartition de tissu,
- le module (25) de mise à disposition est constitué pour mettre à disposition la température et/ou la répartition (T_{A}) de température déterminée,
- le dispositif capteur est constitué pour mettre à disposition la première température et/ou la deuxième température, dans lequel le dispositif capteur comprend un premier élément (11) et un deuxième élément (11) capteur, dans lequel le premier élément (11) capteur et le deuxième élément (11) capteur sont à distance à une distance de capteur, dans lequel la première position (S) de capteur comprend une première position (S_{E1}) de l'élément de capteur pour le premier élément (11) de capteur et une deuxième position (S_{E2}) d'élément de capteur pour le deuxième élément (11) de capteur, dans lequel la première température et/ou la deuxième température comprend une première valeur de température pour le premier élément (11) de capteur et une deuxième valeur de température pour le deuxième élément (11) de capteur.
